# FASCICULE DE BREVET EUROPEEN

(11) **EP 1 824 531 B1**
(45) Date de publication et mention de la délivrance du brevet: **28.05.2008**
(21) Numéro de dépôt: 05817573.8
(22) Date de dépôt: 15.11.2005
(51) Int. Cl.: A61L 27/54, A61L 27/56, A61L 29/16, A61L 29/14, A61L 31/16, A61L 31/14, A61K 47/48, A61K 47/40, C08B 37/00, A61L 27/34, A61L 29/08, A61L 31/10

(54) **BIOMATERIAUX PORTEURS DE CYCLODEXTRINES AUX PROPRIETES D'ABSORPTION AMELIOREES ET DE LIBERATION PROGRESSIVE ET RETARDEE DE MOLECULES THERAPEUTIQUES**
CYCLODEXTRINE TRAGENDE BIOLOGISCHE MATERIALIEN MIT VERBESSERTEN ABSORPTIONSEIGENSCHAFTEN ZUR PROGRESSIVEN UND VERZÖGERTEN FREISETZUNG VON THERAPEUTISCHEN MOLEKÜLEN
BIOMATERIALS CARRYING CYCLODEXTRINES HAVING IMPROVED ABSORPTION PROPERTIES AND USED FOR THE PROGRESSIVE AND DELAYED RELEASE OF THERAPEUTIC MOLECULES

(30) Priorité: 15.11.2004 FR 0412086
(43) Date de publication de la demande: 29.08.2007
(73) Titulaire: Université des Sciences et Technologies de Lille SAIC, 59655 Villeneuve d'Asq (FR)
(72) Inventeur: MARTEL, Bernard, F-59850 Nieppe (FR); BLANCHEMAIN, Nicolas, F-62380 Lumbres (FR); BOSCHIN, François, F-59910 Bondues (FR); HAULON, Stéphan, F-59000 Lille (FR); DELCOURT-DEBRUYNE, Elisabeth, F-59310 Lambersart (FR); MORCELLET, Michel, F-59650 Villeneuve D'ascq (FR); HILDEBRAND, Hartmut, Friedrich, F-59000 Lille (FR)
(74) Mandataire: Hennion, Jean-Claude
(86) Numéro de dépôt international: PCT/FR2005/002829
(87) Numéro de publication internationale: WO 2006/051227

(56) Documents cités:
- EP-B- 1 157 156
- EP-B- 1 165 621
- WO-A-01/85218
- DE-A1- 19 849 464
- US-A- 5 183 809
- US-B1- 6 689 378

## Description

La présente invention est relative à des biomatériaux porteurs de cyclodextrines aux propriétés d'absorption améliorées et de libération progressive et retardée de molécules thérapeutiques, leur procédé de préparation ainsi que leur utilisation notamment à titre de prothèse ou d'implant.

L'implantation de biomatériaux dans un organisme animal ou humain occasionne dans une proportion non négligeable des phénomènes d'infection liés à l'introduction de germes pathogènes lors de l'intervention chirurgicale. Ceci concerne environ 1% des cas dans le domaine de la chirurgie vasculaire, et ces complications peuvent aboutir à la mort du patient dans environ 50% des cas.

Il est possible de combattre ce problème par un traitement curatif à l'aide de biocide (antibiotique ou antiseptique), ou de le combattre de manière prophylactique, en traitant le biomatériau par immersion dans une solution d'antibiotique ou d'antiseptique, juste avant son implantation. Le principe actif est ensuite sensé être libéré *in situ,* directement au sein de la zone sensible. Cependant, cette parade n'est pas infaillible, puisque la nature de la matière qui constitue le biomatériau est telle que :
- la quantité de principe actif réellement adsorbée par celui-ci peut être très faible,
- le principe actif est libéré de manière immédiate dans l'organisme (quelques minutes à quelques heures), et
- sa concentration décroît rapidement en dessous de la concentration minimale inhibitrice (CMI).

La conséquence est que ces systèmes ont une efficacité trop limitée dans le temps par rapport à la durée possible de risque d'infection (quelques semaines).

Différents types de biomatériaux permettant une libération contrôlée (en temps et/ou en quantité) de principes actifs ont été proposés.

Dans certains cas, il s'agit de biomatériaux constitués de matrices polymères comportant des pores, à l'intérieur desquels est contenu le principe actif. La libération du principe actif se fait par diffusion à l'extérieur des pores de la matrice.

A cette fin, le document WO 02/41928 décrit l'utilisation d'un biomatériau à base de polymère poreux constitué d'un réseau polymère hydrophile ou amphiphile (réseau support) dont les pores renferment un polymère gélifié contenant un ou plusieurs principes actifs (réseau de remplissage). De préférence, le réseau support est une microsphère poreuse formée de copolymères acryliques modifiés par de groupements diéthylaminoéthyl. L'exemple 3 de ce document illustre l'étude comparative de la cinétique de libération de l'indométacine, de différentes microsphères conformes à l'invention chargées en indométacine. Les résultats présentés dans le tableau II de ce document montrent que, bien qu'il y ait une libération progressive de la quantité de principe actif, le retard par rapport aux microsphères témoins (contenant le principe actif dans le pores du réseau support) n'est pas significatif.

Dans d'autres cas, il s'agit de biomatériaux constitués de polymères biodégradables qui libèrent le(s) principe(s) actif(s) inclus dans la matrice polymère au fur et à mesure que celle-ci se dégrade et se résorbe dans le corps. La vitesse de libération du principe actif dépend de la vitesse de dégradation de la matrice.

Ainsi, le document US 6.525.145 décrit un copolymère biodégradable formé de polylactate et d'un polysaccharide tel que le dextran ; préalablement à leur copolymérisation, au moins un principe actif est lié de manière covalente au dextran. Dans une variante de réalisation un second principe actif peut être incorporé de manière non covalente au copolymère ; ce second principe actif peut être libéré par deux mécanismes différents :
- par diffusion, avant la dégradation du copolymère ;
- par libération de la matrice polymère au fur et à mesure que le copolymère se dégrade.

Selon les auteurs, la libération du ou des principe(s) actif(s) de ce biomatériau peut être modulée en fonction de plusieurs critères, à savoir :
- la nature (hydrophile ou non) du principe actif lié de façon covalente au dextran ;
- l'hydrolyse de la molécule de dextran, qui est fonction :
   o de la nature de ses liaisons glycosidiques (vitesse d'hydrolyse plus élevée pour les liaisons 1,6 par rapport aux liaisons 1,4) ;
   o de la quantité de dextran incorporé dans le copolymère.

Cependant, aucune étude de cinétique de libération du principe actif n'étant incluse dans ce document, il est difficile d'apprécier l'efficacité de ce système de libération contrôlée.

La présente invention se propose de pallier les inconvénients présentés par les différents types de biomatériaux connus aptes à libérer progressivement un principe actif.

L'invention a pour objet des biomatériaux porteurs de cyclodextrines qui présentent une capacité des relargage améliorée, en termes de quantité totale d'agents bioactifs libérés, en termes de libération progressive et en termes de durée totale de libération de l'agent bioactif. Cette invention fait appel au principe du greffage du biomatériau par la cyclodextrine, de façon à ce que celle-ci y adhère de manière quasi permanente, via des liaisons covalentes ou par interactions physiques.

A cet effet et selon un premier aspect, l'invention a pour objet un procédé de préparation d'un biomatériau comportant au moins une molécule bioactive à partir d'un biomatériau de base caractérisé par les opérations successives suivantes effectuées sur ledit biomatériau de base:
a) application d'un mélange solide de :
   - cyclodextrine(s) et/ou de dérivé(s) de cyclodextrine(s) et/ou de complexe(s) d'inclusion de cyclodextrine(s) et/ou de dérivés de cyclodextrine(s),
   - d'au moins un acide poly(carboxylique),
   - et éventuellement d'un catalyseur;
b) chauffage à une température comprise entre 100°C et 220°C, pendant une durée de 1 à 60 minutes ;
c) lavage à l'eau;
d) séchage,
et en ce qu'au moins un agent bioactif est incorporé au biomatériau, par imprégnation du biomatériau après l'étape de séchage dans une solution concentrée d'agent bioactif.

Selon un deuxième aspect, l'invention concerne un biomatériau préparé à partir d'un biomatériau de base présentant une structure chimique qui comporte une fonction hydroxyle et/ou une fonction amine, ladite structure de base étant liée de façon covalente à au moins une molécule de cyclodextrine ou à un polymère composé de cyclodextrine dont la structure comporte la répétition du motif de formule générale : 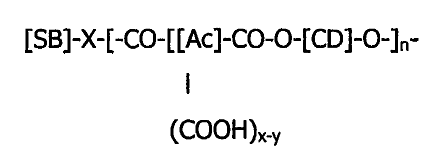 SB représentant la structure du biomatériau de base constitué d'un matériau polymère d'origine naturelle ou artificielle et/ou d'un matériau biocéramique, X étant soit un atome d'oxygène faisant partie d'un groupe ester, soit un groupe NH,
x ≥ 3, et 2≤y ≤(x-1) et (x-y) ≥1,
- x étant le nombre de fonctions acide carboxylique portées par l'acide poly(carboxylique) avant réaction,
- y représente le nombre de fonctions acide carboxyliques de l'acide poly(carboxylique) estérifiées ou amidifiées lors de la réaction,
- (x-y) est le nombre de fonctions acide carboxyliques de l'acide poly(carboxylique) non estérifiées ou amidifiées après réaction,
- 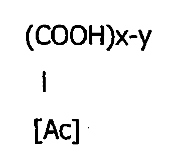 représentant la chaîne moléculaire d'un acide poly(carboxylique) de formule 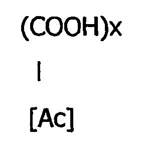 dont au moins deux fonctions carboxyle (-COOH) ont subi une estérification ou subi respectivement une estérification et une amidification et qui porte au moins une fonction carboxyle n'ayant pas subi de réaction d'estérification ou d'amidification ;
- [CD] représentant la structure moléculaire des cyclodextrines choisies préférentiellement parmi : l'α-cyclodextrine, la β-cyclodextrine, la γ-cyclodextrine et leurs mélanges; leurs dérivés qui sont choisis préférentiellement parmi les dérivés aminés, méthylés, hydroxypropylés, sulfobutylés, carboxyméthylés, ou carboxyliques, et leurs mélanges ; les complexes d'inclusion desdites cyclodextrines ou desdits dérivés de cyclodextrine avec des molécules bioactives,
caractérisé en ce que, ledit biomatériau comportant au moins un agent bioactif formant un complexe avec les molécules de cyclodextrine et/ou de dérivés de cyclodextrine, ledit agent bioactif est une molécule thérapeutique.

Selon un troisième aspect, l'invention concerne un biomatériau préparé à partir d'un biomatériau de base dont la structure poreuse est occupée, ou la surface de la structure est enrobée ou recouverte par un polymère réticulé de cyclodextrine(s) et/ou de dérivés de cyclodextrine(s) et/ou de complexes d'inclusion de cyclodextrine ou de dérivés de cyclodextrines et dont la structure comporte la répétition d'un motif de formule générale : 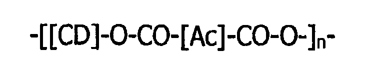 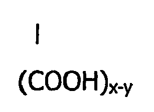 avec x ≥ 3, et 2≤y ≤(x-1) et (x-y) ≥1,
- x étant le nombre de fonctions carboxyle portées par l'acide poly(carboxylique) avant réaction,
- y représentant le nombre de fonctions carboxyle de l'acide poly(carboxylique) estérifiées lors de la réaction,
- (x-y) étant le nombre de fonctions carboxyle de l'acide poly(carboxylique) non estérifiées après réaction
- 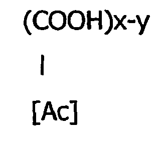 représentant la chaîne moléculaire d'un acide poly(carboxylique) de formule : 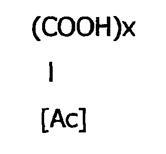 dont au moins deux fonctions carboxyle (-COOH) ont subi une estérification et qui porte au moins une fonction carboxyle n'ayant pas subi de réaction d'estérification;
- [CD] représentant la structure moléculaire des cyclodextrines choisies préférentiellement parmi: l'α-cyclodextrine, la β-cyctodextrine, la γ-cyclodextrine et leurs mélanges; leurs dérivés qui sont choisis préférentiellement parmi les dérivés aminés, méthyles, hydroxypropylés, sulfobutylés, carboxyméthylés, ou carboxyliques, et leurs mélanges ; les complexes d'inclusion desdites cyclodextrines ou desdits dérivés de cyclodextrine avec des molécules bioactives,
caractérisé en ce que, ledit biomatériau comportant au moins un agent bioactif formant un complexe avec les molécules de cyclodextrine et/ou de dérivés de cyclodextrine, ledit agent bioactif est une molécule thérapeutique.

Selon un quatrième aspect, l'invention se rapporte à l'utilisation d'un biomatériau tel que défini précédemment à titre d'implant, prothèse ou dispositif pour la libération contrôlée d'au moins une molécule thérapeutique.

L'invention va maintenant être décrite en détail.

La présente invention concerne un nouveau type de biomatériaux porteurs de cyclodextrines complexées avec au moins un agent bioactif, qui présentent une capacité de relargage améliorée, en termes de quantité totale d'agent bioactif libéré, en terme de libération progressive et en terme de durée totale de libération de l'agent bioactif.

Cette nouvelle fonctionnalité a pour but de réduire sensiblement les risques d'infections post-opératoires liés à la colonisation de la plaie ou de la greffe par des bactéries pathogènes, pendant une période critique qui peut s'étaler jusqu'à 6 à 8 semaines.

Ces conditions justifient par conséquent la conception d'un biomatériau capable d'absorber une quantité suffisante d'agent bioactif et qui puisse ensuite être libérée progressivement pendant la totalité de cette période.

L'invention fait appel au principe du greffage du biomatériau de base par la cyclodextrine (CD), de façon à ce que celle-ci y adhère de manière quasi permanente, via des liaisons covalentes ou par interactions physiques. Dans ce cas la cyclodextrine fait partie intégrante du biomatériau de base.

Dans un mode de réalisation, au moins un agent bioactif est incorporé dans un second temps au biomatériau de base greffé, par imprégnation dans une solution concentrée d'agent bioactif, opération pendant laquelle il vient s'inclure à l'intérieur des cavités de la cyclodextrine présente sur le biomatériau.

Dans un autre mode de réalisation, le complexe d'inclusion cyclodextrine - agent bioactif est formé dans un premier temps, puis celui-ci est greffé sur le biomatériau de base, dans un second temps.

Par "biomatériau" on entend tout matériau non vivant, d'origine naturelle ou artificielle, toléré par l'organisme humain ou animal, ayant une durée de contact avec l'organisme supérieure à plusieurs semaines, utilisé dans l'élaboration d'un dispositif médical destiné à être mis en contact avec des tissus biologiques (peau, dent, os, sang, ...) et visant à remplacer, ou traiter, un tissu, un organe ou une fonction.

Le biomatériau de base est un biomatériau qui ne comporte pas de molécules bioactives.

La fixation des molécules de cyclodextrine(s) sur le biomatériau de base est principalement réalisée selon deux mécanismes qui dépendent chacun de la nature chimique de ce biomatériau.

Dans le cas du traitement de biomatériaux de base présentant une structure chimique qui comporte une fonction hydroxyle et/ou amine, le procédé de l'invention permet dans un premier temps, de former un anhydride de l'acide poly(carboxylique) qui réagit avec le biomatériau en formant une liaison covalente de type amide ou ester entre le biomatériau traité et l'acide poly(carboxylique). Ensuite, dans le cas de figure le plus simple, un second anhydride de l'acide poly(carboxylique) déjà lié au biomatériau est formé, celui-ci réagit avec une molécule de cyclodextrine ou de dérivé de cyclodextrine en créant une liaison ester avec la molécule de cyclodextrine ou de dérivé de cyclodextrine.

Par ailleurs, certains biomatériaux polymères synthétiques ou minéraux ne possèdent pas de groupes fonctionnels aptes à réagir selon le mécanisme proposé ci-dessus. Dans ce cas, la fixation de cyclodextrine(s) et/ou de dérivés de cyclodextrine(s) est réalisée par la formation d'un polymère réticulé obtenu par réaction exclusive entre les molécules de cyclodextrine(s) et/ou de dérivé de cyclodextrine(s) et au moins un acide poly(carboxylique). Le polymère réticulé ainsi formé forme un film ou un dépôt en surface du biomatériau de base ou est confiné à l'intérieur de sa structure poreuse et y reste fixé de façon permanente, selon la structure du biomatériau traité.

Lorsque le polymère : 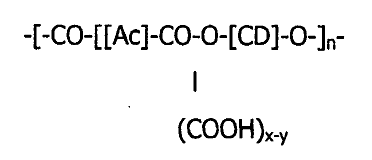 se forme à partir d'une molécule de cyclodextrine fixée à la structure du biomatériau de base par liaison covalente, il présente au moins une liaison covalente avec le biomatériau de base.

Lorsque le copolymère : 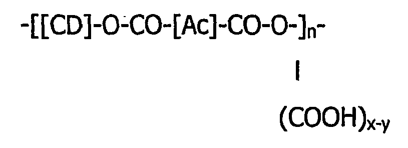 se forme à partir de molécules d'acide poly(carboxylique) et de cyclodextrine et/ou de dérivé(s) de cyclodextrine(s) non liées à la structure de base du biomatériau il peut néanmoins, s'il est réticulé, c'est-à-dire qu'il forme un réseau tridimensionnel entremêlant ou enrobant la structure du biomatériau de base, être fixé, physiquement, de manière permanente au biomatériau considéré.

Le mécanisme de base mettant en jeu une molécule d'acide poly(carboxylique) et une molécule de cyclodextrine ou de dérivé de cyclodextrine a été décrit par le demandeur dans les brevets antérieurs EP 1 165 621 B1 et EP 1 157 156 B1.

Dans le cas d'un biomatériau polymère de base comportant une fonction amine ou hydroxyle comme par exemple, le chitosane, la kératine ou la cellulose et ses dérivés, les deux mécanismes de fixation, à savoir la fixation par une liaison covalente et la formation d'un film, d'un enrobage polymère réticulé sur le biomatériau coexistent.

Selon un mode de réalisation préféré, l'application du mélange solide est obtenue par imprégnation du matériau avec une solution aqueuse:
- de cyclodextrine(s) et/ou de dérivé(s) de cyclodextrine(s) ou de complexes de cyclodextrines, ou de complexes d'inclusion cyclodextrine - agent bioactif,
- d'au moins un acide poly(carboxylique),
- et éventuellement d'un catalyseur
puis séchage du biomatériau de base imprégné.

Cette imprégnation et ce séchage permettent de déposer uniformément les réactifs en surface du biomatériau de base ou à l'intérieur de ses pores ce qui facilite ultérieurement, à la fois la réaction de fixation de la cyclodextrine et l'obtention d'un dépôt ou enrobage uniforme de polymère en surface ou dans les pores du biomatériau de base.

Selon une variante préférée, le biomatériau de base est séché à une température comprise entre 40°C et 90°C, de préférence, avant l'opération de chauffage proprement dite réalisée à une température comprise entre 100°C et 220°C, pendant une durée variant de 1 à 60 minutes.

Le chauffage proprement dit est destiné à la fixation permanente des molécules de cyclodextrine(s) sur le biomatériau de base, par réaction entre l'acide poly(carboxylique) et le biomatériau de base, selon au moins un des mécanismes suivants :
- greffage chimique par liaison covalente entre la structure du biomatériau de base et la molécule de cyclodextrine ou de dérivé de cyclodextrine éventuellement complexée avec au moins une molécule bioactive, voire de polymère de cyclodextrine(s) et d'acide(s) poly(carboxylique)(s) ;
- réaction entre l'acide poly(carboxylique) et la cyclodextrine et/ou le dérivé de cyclodextrine(s) ou de leurs complexes d'inclusion avec des molécules bioactives pour former un copolymère réticulé (greffage physique par enrobage).

De préférence, l'acide polycarboxylique est choisi parmi les acides poly(carboxyliques) acycliques saturés et insaturés cycliques saturés et insaturés aromatiques, les acides hydroxypoly(carboxyliques), de préférence, l'acide citrique, l'acide polyacrylique, l'acide poly(méthacrylique), l'acide 1,2,3,4-butanetétracarboxylique, l'acide maléique, l'acide citraconique, l'acide itaconique, l'acide 1,2,3-propanetricarboxylique, l'acide trans-aconitique, l'acide all-cis-1,2,3,4-cydopentanetétracarboxylique, l'acide méllittique, l'acide oxydisuccinique, l'acide thiodisuccinique.

On pourra aussi utiliser les anhydrides dérivés des acides sus nommés.

De préférence, le mélange contient un catalyseur choisi parmi les dihydrogénophosphates, les hydrogénophosphates, les phosphates, les hypophosphites, les phosphites de métaux alcalins, les sels de métaux alcalins des acides polyphosphoriques, les carbonates, les bicarbonates, les acétates, les borates, les hydroxydes de métaux alcalins, les amines aliphatiques et l'ammoniaque, et de préférence, parmi l'hydrogénophosphate de sodium, le dihydrogénophosphate de sodium et l'hypophosphite de sodium.

De préférence, la cyclodextrine est choisie parmi : l'α-cyclodextrine, la β-cyclodextrine, la γ-cyclodextrine et leurs mélanges ; les dérivés de cyclodextrine, choisis parmi les dérivés aminés, méthylés, hydroxypropylés, sulfobutylés, carboxyméthylés, ou carboxyliques de l'α-cyclodextrine, de la β-cyclodextrine et de la γ-cyclodextrine, et leurs mélanges ; et les complexes d'inclusion desdites cyclodextrines ou desdits dérivés de cyclodextrine avec des molécules bioactives.

Selon un deuxième aspect, l'invention concerne un biomatériau préparé, de préférence, par le procédé selon l'invention à partir d'un biomatériau de base présentant une structure chimique qui comporte une fonction hydroxyle et/ou une fonction amine, ladite structure de base étant liée de façon covalente à au moins une molécule de cyclodextrine ou à un polymère composé de cyclodextrine dont la structure comporte la répétition du motif de formule générale : 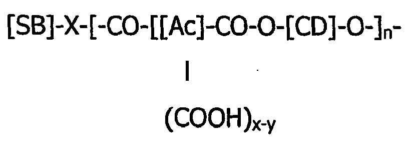 SB représentant la structure du biomatériau de base constitué d'un matériau polymère d'origine naturelle ou artificielle et/ou d'un matériau biocéramique ou d'un polyol, X étant un atome d'oxygène ou un groupe NH,
x ≥ 3, et 2≤y ≤(x-1) et (x-y) ≥1,
- x étant le nombre de fonctions carboxyle portées par l'acide poly(carboxylique) avant réaction,
- y représente le nombre de fonctions carboxyle de l'acide poly(carboxylique) estérifiées ou amidifiées lors de la réaction,
- (x-y) est le nombre de fonctions carboxyle de l'acide poly(carboxylique) non estérifiées ou amidifiées après réaction,
- 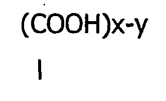 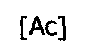 représentant la chaîne moléculaire d'un acide poly(carboxylique) de formule 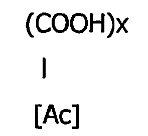 dont au moins deux fonctions carboxyle (-COOH) ont subi une estérification ou subi respectivement une estérification et une amidification et qui porte au moins une fonction carboxyle n'ayant pas subi de réaction d'estérification ou d'amidification ;
- [CD] représentant la structure moléculaire des cyclodextrines choisies préférentiellement parmi : l'α-cyclodextrine, la β-cyclodextrine, la γ-cyclodextrine et leurs mélanges; leurs dérivés qui sont choisis préférentiellement parmi les dérivés aminés, méthylés, hydroxypropylés, sulfobutylés, carboxyméthylés, ou carboxyliques et leurs mélanges; les complexes d'inclusion desdites cyclodextrines ou desdits dérivés de cyclodextrine avec des molécules bioactives,
caractérisé en ce que, ledit biomatériau comportant au moins un agent bioactif formant un complexe avec les molécules de cyclodextrine et/ou de dérivés de cyclodextrine, ledit agent bioactif est une molécule thérapeutique.

Par "molécule thérapeutique" on entend toute molécule destinée à la prophylaxie et/ou au traitement des maladies chez l'homme ou l'animal.

Selon un troisième aspect, l'invention concerne un biomatériau préparé, de préférence, par le procédé selon l'invention à partir d'un biomatériau de base dont la structure poreuse est occupée, ou la surface de la structure est enrobée ou recouverte par un polymère réticulé de cyclodextrine(s) et/ou de dérivés de cyclodextrine(s) et/ou de complexes d'inclusion de cyclodextrine ou de dérivés de cyclodextrines et dont la structure comporte la répétition d'un motif de formule générale : 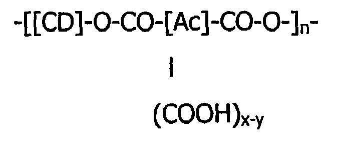 avec x ≥ 3, et 2≤y ≤(x-1) et (x-y) ≥1,
- x étant le nombre de fonctions carboxyle portées par l'acide poly(carboxylique) avant réaction,
- y représentant le nombre de fonctions carboxyle de l'acide poly(carboxylique) estérifiées lors de la réaction,
- (x-y) étant le nombre de fonctions carboxyle de l'acide poly(carboxylique) non estérifiées après réaction ;
- 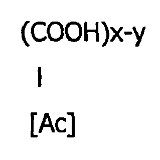 représentant la chaîne moléculaire d'un acide poly(carboxylique) de formule : 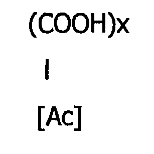 dont au moins deux fonctions carboxyle (-COOH) ont subi une estérification et qui porte au moins une fonction carboxyle n'ayant pas subi de réaction d'estérification;
- [CD] représentant la structure moléculaire des cyclodextrines choisies préférentiellement parmi: l'α-cyclodextrine, la β-cyctodextnne, la γ-cyclodextrine et leurs mélanges; leurs dérivés qui sont choisis préférentiellement parmi les dérivés aminés, méthylés, hydroxypropylés, sulfobutylés, carboxyméthylés, ou carboxyliques et leurs mélanges; les complexes d'inclusion desdites cyclodextrines ou desdits dérivés de cyclodextrine avec des molécules bioactives,
caractérisé en ce que, ledit biomatériau comportant au moins un agent bioactif formant un complexe avec les molécules de cyclodextrine et/ou de dérivés de cyclodextrine, ledit agent bioactif est une molécule thérapeutique.

Ainsi, la présente invention concerne les biomatériaux préparés à partir de biomatériaux de base sur lesquels des molécules de cyclodextrine(s) et/ou de dérivés de cyclodextrine(s) sont uniquement fixées par liaison covalente, les biomatériaux préparés à partir de biomatériaux de base sur lesquels des molécules de cyclodextrine(s) et/ou de dérivés de cyclodextrine(s) sont fixées à la fois par liaison covalente et par occupation de la structure poreuse, ou par enrobage ou revêtement de la surface de la structure du biomatériau de base par un polymère réticulé de cyclodextrine(s), et les biomatériaux préparés à partir de biomatériaux de base sur lesquels la cyclodextrine est uniquement fixée par occupation de la structure poreuse, ou par enrobage ou revêtement de la surface de la structure du biomatériau de base par un polymère réticulé de cyclodextrine(s).

Le biomatériau peut être fonctionnalisé avec un agent bioactif selon l'invention par différents mécanismes.

Dans une variante de réalisation, le biomatériau de base est greffé avec les molécules de cyclodextrine(s) et/ou de dérivés de cyclodextrine(s) selon l'un ou l'autre des modes de réalisation décrits plus haut, puis il est imprégné dans une solution concentrée de l'agent bioactif. Cette opération a pour but de mettre en présence la cyclodextrine greffée et l'agent bioactif, et il en résulte la formation du complexe d'inclusion de type *hote-invitée*. Ainsi on obtient un biomatériau chargé en principe actif qui sera libéré ensuite de manière ralentie une fois celui-ci implanté dans l'organisme.

Dans un autre mode de réalisation, le biomatériau de base est greffé avec le complexe d'inclusion cyclodextrine - agent bioactif ou dérivé de cyclodextrine - agent bioactif qui aura préalablement été préparé selon les techniques de l'art du métier. Ainsi dans le procédé de fabrication, il suffit de remplacer la cyclodextrine par le complexe de cyclodextrine - agent bioactif.

La présente invention permet de tirer partie des qualités de complexation de la cyclodextrine en greffant cette dernière sur le biomatériau de base, par l'établissement d'une liaison chimique covalente, ou par dépôt d'un polymère de cyclodextrine qui adhérerait au biomatériau par interactions physiques.

Le greffage chimique ou physique de la cyclodextrine sur le biomatériau de base présenterait l'avantage que celle-ci reste présente sur ce dernier, et n'en soit pas détachée par dissolution immédiate dans le milieu physiologique dans lequel serait implanté le biomatériau.

Les biomatériaux faisant l'objet de l'invention présentent la capacité d'absorber de manière accrue et à libérer de manière prolongée des substances bioactives telles que des anticoagulants, des anti-thrombogéniques, des agents anti-mitotiques, des agents anti-prolifération, anti-adhésion, anti-migration, des promoteurs d'adhésion cellulaire, des facteurs de croissance, des molécules antiparasitaires, des anti-inflammatoires, des angiogéniques, des inhibiteurs de l'angiogenèse, des vitamines, des hormones, des protéines, des antifongiques, des molécules antimicrobiennes, des antiseptiques ou des antibiotiques.

L'effet de capacité accrue d'absorption des molécules bioactives provient du fait que les cyclodextrines greffées sont des sites de complexation qui complexent activement ces molécules bioactives, contrairement aux surfaces non greffées qui ne présentent que des interactions non spécifiques avec les substrats (liaisons hydrogène ioniques et de Van der Waals).

L'effet de libération retardée provient de l'action de la cyclodextrine qui a la propriété de former des complexes d'inclusion de type *hote-invitée* avec les molécules précitées, et de libérer l'invitée de manière ralentie et progressive.

Par ailleurs, il est connu qu'un même agent thérapeutique présente des constantes d'équilibre de complexation différentes, en fonction de la nature des cyclodextrines utilisées (alpha, beta, gamma, hydroxypropyl alpha, etc) ; de ce fait, chaque type de cyclodextrine fixé sur le biomatériau de base va libérer l'agent thérapeutique selon une cinétique propre, plus ou moins rapide. Par conséquent, il sera possible de moduler la cinétique de libération d'un agent bioactif, en utilisant un mélange de plusieurs cyclodextrines lors du greffage sur le biomatériau de base et en ajustant la proportion de chaque cyclodextrine dans ce mélange.

En outre, compte tenu de la présence des fonctions carboxyliques (ou carboxylates, selon le pH du milieu) sur un biomatériau selon l'invention, celles-ci peuvent être utilisées en tant qu'échangeurs d'ions. On peut ainsi substituer l'ion H⁺ d'une fonction carboxylique (ou Na⁺ d'un carboxylate) par un autre cation, chois par exemple dans le groupe comprenant des ions d'argent, les ions ammonium quaternaire, le chlorhydrate de chlorhexidine, tous connus pour leurs propriétés biocides.

L'imprégnation du biomatériau selon l'invention d'une solution d'un composé biocide cationique (réalisée après l'étape de séchage) permet de conférer audit biomatériau des propriétés biocides, le biomatériau fixant l'agent biocide par un mécanisme d'échange ionique. Le biomatériau ainsi traité présentera des propriétés supplémentaires, distinctes ou non de celles déjà conférées par l'agent thérapeutique complexé par les cyclodextrines. L'homme du métier pourra décider, en fonction de l'agent thérapeutique à libérer ou de l'agent biocide à fixer, d'utiliser l'une ou l'autre de ces fonctions, ou bien de les utiliser en même temps. Ce dernier cas de figure correspond par exemple au chlorhydrate de chlorhexidine, composé qui est à la fois aromatique (donc apte à être inclus dans les cyclodextrines) et cationique (donc apte à être fixé sur les groupes carboxyles). Dans ce cas, le même agent bioactif sera libéré in vivo selon deux mécanismes différents.

Selon un quatrième aspect, l'invention se rapporte à l'utilisation d'un biomatériau tel que défini précédemment à titre d'implant, prothèse ou dispositif pour la libération contrôlée d'au moins une molécule thérapeutique.

La présente invention concerne l'utilisation des biomatériaux, porteurs de cyclodextrine complexée avec au moins un agent bioactif, à titre de prothèses et endoprothèses vasculaires (stents couverts), de plaques de contention d'hernies, de membranes de régénération tissulaire guidée, de régénération osseuse guidée, ou de dispositifs de libération intrasucculaires, de tubes et cathéters de dialyse, de perfusion, de transfusion, de nutrition artificielle, d'implants transcutanés, de treillis et réseaux pour ingénierie tissulaire, de substituts osseux micro et macroporeux, de fils de suture et de pansements à usage médical ou vétérinaire.

La structure du biomatériau de base selon l'invention peut être constituée de matière polymère, telles que le polytéréphtalate d'éthylène glycol (PET), l'acide polylactique (PLA), l'acide polyglycolique (PGA) et leurs copolymères (PLA-GA), le fluorure de polyvinylidène (PVDF), le polytétrafluoroéthylène (PTFE), la cellulose, la cellulose oxydée, la cellulose régénérée, le polyéthylène glycol (PEG), le polyamide-6, le polyamide-6,6, le polypropylène, le polyéthylène, les polysaccharides tels que pectine, carraghénanes, alginates et dextranes, la kératine, le chitosane, le collagène ou la gélatine ou de la combinaison de ces polymères.

Dans d'autres modes de réalisation, la structure du biomatériau de base selon l'invention est constituée de composés minéraux tels que les biocéramiques : phosphates de calcium, hydroxyapatites, alumine, zircone, verres, verres ionomères...

Dans d'autres modes de réalisation, la structure du biomatériau de base selon invention est constituée de composites combinant des composés minéraux et des matières polymères telles que décrites ci-dessus.

L'invention a pour objet également des dispositifs pour la libération contrôlée d'au moins une molécule thérapeutique comprenant au moins un biomatériau tel que décrit ci-dessus.

L'invention concerne aussi des compositions renfermant au moins un biomatériau tel que défini ci-dessus.

La présente invention sera mieux comprise à la lecture des exemples suivants qui sont donnés, de façon non limitative, en vue de mieux illustrer les caractéristiques des biomatériaux qui font l'objet de la présente invention.

### EXEMPLE 1

Un échantillon de prothèse vasculaire en PET tissé a été imprégné à l'aide d'un foulard d'une solution aqueuse contenant de la β-CD (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 30 minutes à 140°, lavé par soxhlet à l'eau, et séché. Le gain de poids lié au greffage a été de 2,4%.

### EXEMPLE 2

Un échantillon de prothèse vasculaire en PET tissé a été imprégné à l'aide d'un foulard d'une solution aqueuse contenant de la β-CD (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 30 minutes à 150°C, lavé par soxhlet à l'eau, et séché. Le gain de poids lié au greffage a été de 7,4%.

### EXEMPLE 3

Un échantillon de prothèse vasculaire en PET tissé a été imprégné à l'aide d'un foulard d'une solution aqueuse contenant de la β-CD (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 30 minutes à 160°C, lavé par soxhlet à l'eau, et séché. Le gain de poids lié au greffage a été de 9,2%.

### EXEMPLE 4

Un échantillon de prothèse vasculaire en PET tissé a été imprégné à l'aide d'un foulard d'une solution aqueuse contenant de la β-CD (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 30 minutes à 170°C, lavé par soxhlet à l'eau, et séché. Le gain de poids lié au greffage a été de 9,9%.

### EXEMPLE 5

Un échantillon de prothèse vasculaire en PET tissé a été imprégné à l'aide d'un foulard d'une solution aqueuse contenant de la β-CD (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 5 minutes à 170°C, lavé par soxhlet à l'eau, et séché. Le gain de poids lié au greffage a été de 8,8%.

### EXEMPLE 6

Un échantillon de prothèse vasculaire en PET tissé a été imprégné à l'aide d'un foulard d'une solution aqueuse contenant de la β-CD (100 g/L), d'acide 1,2,3,4-butanetétracarboxylique (80 g/L) et de l'hypophosphite de sodium (10 g/L). Le taux d'emport était de 70%. L'échantillon a ensuite été séché pendant 7 minutes à 90°C, puis traité pendant 6 minutes à 175°C, lavé par soxhlet à l'eau, et séché. Le gain de poids lié au greffage a été de 25%.

### EXEMPLE 7

Un échantillon de prothèse vasculaire en PET tissé a été imprégné à l'aide d'un foulard d'une solution aqueuse contenant de la hydroxypropyl-bêta-cyclodextrine (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 10 minutes à 140°C, lavé par soxhlet à l'eau, et séché. Le gain de poids lié au greffage a été de 5%.

### EXEMPLE 8

Un échantillon de prothèse vasculaire en PET tissé a été imprégné à l'aide d'un foulard d'une solution aqueuse contenant de la hydroxypropyl-bêta-cyclodextrine (100 g/L), de l'acide citrique (80 g/L) et de l'hypophosphite de sodium (10 g/L). Le taux d'emport était de 70%. L'échantillon a ensuite été séché pendant 7 minutes à 104°C, puis traité pendant 10 minutes à 150°C, lavé par soxhlet à l'eau, et séché. Le gain de poids lié au greffage a été de 11,1%.

### EXEMPLE 9

Un échantillon de prothèse vasculaire en PET tissé a été imprégné à l'aide d'un foulard d'une solution aqueuse contenant de la hydroxypropyl-gamma-cyclodextrine (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 10 minutes à 160°C, lavé par soxhlet à l'eau, et séché. Le gain de poids lié au greffage a été de 10%.

### EXEMPLE 10

Un échantillon de prothèse vasculaire en PET tissé a été imprégné à l'aide d'un foulard d'une solution aqueuse contenant de la hydroxypropyl-gamma-cyclodextrine (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 5 minutes à 150°C, lavé par soxhlet à l'eau, et séché. Le gain de poids lié au greffage a été de 6,5%.

### EXEMPLE 11

Un échantillon de prothèse vasculaire en PET tissé a été imprégné à l'aide d'un foulard d'une solution aqueuse contenant de la hydroxypropyl-gamma-cyclodextrine (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 10 minutes à 150°C, lavé par soxhlet à l'eau, et séché. Le gain de poids lié au greffage a été de 8,1%.

### EXEMPLE 12

Un échantillon de prothèse vasculaire en PET tissé a été imprégné à l'aide d'un foulard d'une solution aqueuse contenant de la hydroxypropyl-gamma-cyclodextrine (100 g/L), de l'acide citrique (80 g/L) et de l'hypophosphite de sodium (10 g/L). Le taux d'emport était de 70%. L'échantillon a ensuite été séché pendant 7 minutes à 90°C, puis traité pendant 15 minutes à 150°C, lavé par soxhlet à l'eau, et séché. Le gain de poids lié au greffage a été de 11%.

### EXEMPLE 13

Une membrane microporeuse de PVDF a été imprégnée à l'aide d'un foulard d'une solution aqueuse contenant de la β-CD (100 g/L), de l'acide citrique (100 g/L) et de l'hypophosphite de sodium (30 g/L). Le taux d'emport était de 60%. L'échantillon a ensuite été séché pendant 3 minutes à 90°C, puis traité pendant 10 minutes à 140°C, lavé par soxhlet à l'eau, et séché. Le gain de poids lié au greffage a été de 2,4%.

### EXEMPLE 14

Une membrane microporeuse de PVDF a été imprégnée à l'aide d'un foulard d'une solution aqueuse contenant de la β-CD (100 g/L), de l'acide citrique (100 g/L) et de l'hypophosphite de sodium (30 g/L). Le taux d'emport était de 86%. L'échantillon a ensuite été séché pendant 3 minutes à 90°C, puis traité pendant 10 minutes à 150°C, lavé par soxhlet à l'eau, et séché. Le gain de poids lié au greffage a été de 10,5%.

### EXEMPLE 15

Une membrane microporeuse de PVDF a été imprégnée à l'aide d'un foulard d'une solution aqueuse contenant de la β-CD (100 g/L), de l'acide citrique (100 g/L) et de l'hypophosphite de sodium (30 g/L). Le taux d'emport était de 78%. L'échantillon a ensuite été séché pendant 3 minutes à 90°C, puis traité pendant 10 minutes à 170°C, lavé par soxhlet à l'eau, et séché. Le gain de poids lié au greffage a été de 15,5 %.

### EXEMPLE 16

Une membrane microporeuse de PVDF a été imprégnée à l'aide d'un foulard d'une solution aqueuse contenant de la hydroxypropyl-bêta-cyclodextrine (100 g/L), de l'acide citrique (100 g/L) et de l'hypophosphite de sodium (30 g/L). Le taux d'emport était de 85%. L'échantillon a ensuite été séché pendant 3 minutes à 90°C, puis traité pendant 10 minutes à 170°C, lavé par soxhlet à l'eau, et séché. Le gain de poids lié au greffage a été de 15,8%.

### EXEMPLE 17

Une membrane microporeuse de cellulose régénérée a été imprégnée à l'aide d'un foulard d'une solution aqueuse contenant de la β-CD (100 g/L), de l'acide citrique (100 g/L) et de l'hypophosphite de sodium (30 g/L). Le taux d'emport était de 163%. L'échantillon a ensuite été séché pendant 3 minutes à 90°C, puis traité pendant 10 minutes à 170°C, lavé par soxhlet à l'eau, et séché. Le gain de poids lié au greffage a été de 3,95%.

### EXEMPLE 18

Une membrane microporeuse de cellulose régénérée a été imprégnée à l'aide d'un foulard d'une solution aqueuse contenant de la β-CD (100 g/L), de l'acide citrique (100 g/L) et de l'hypophosphite de sodium (30 g/L). Le taux d'emport était de 187%. L'échantillon a ensuite été séché pendant 3 minutes à 90°C, puis traité pendant 10 minutes à 150°C, lavé par soxhlet à l'eau, et séché. Le gain de poids lié au greffage a été de 14,9%.

### EXEMPLE 19

Un échantillon de tissu en polyamide-6 a été imprégné à l'aide d'un foulard d'une solution aqueuse contenant de la β-CD (100 g/L), de l'acide citrique (100 g/L) et de l'hypophosphite de sodium (30 g/L). Le taux d'emport était de 70%. L'échantillon a ensuite été séché pendant 3 minutes à 104°C, puis traité pendant 3 minutes à 170°C, lavé par soxhlet à l'eau, et séché. Le gain de poids lié au greffage a été de 5%.

### EXEMPLE 20

Un échantillon de tissu en polyamide-6 a été imprégné à l'aide d'un foulard d'une solution aqueuse contenant de la β-CD (100 g/L), de l'acide citrique (100 g/L) et de l'hypophosphite de sodium (30 g/L). Le taux d'emport était de 70%. L'échantillon a ensuite été séché pendant 3 minutes à 104°C, puis traité pendant 5 minutes à 170°C, lavé par soxhlet à l'eau, et séché. Le gain de poids lié au greffage a été de 10%.

### EXEMPLE 21

Un échantillon de tissu en polyamide-6 a été imprégné à l'aide d'un foulard d'une solution aqueuse contenant de la β-CD (100 g/L), de l'acide citrique (100 g/L) et de l'hypophosphite de sodium (30 g/L). Le taux d'emport était de 70%. L'échantillon a ensuite été séché pendant 3 minutes à 104°C, puis traité pendant 12 minutes à 170°C, lavé par soxhlet à l'eau, et séché. Le gain de poids lié au greffage a été de 12,5%.

### EXEMPLE 22

Cet exemple illustre les capacités d'absorption accrue et de libération retardée d'une membrane de PVDF fonctionnalisée par la β-CD vis-à-vis de la chlorhexidine, antiseptique choisi comme principe actif. Une membrane vierge et une seconde fonctionnalisée par la β-CD ont été plongées dans un premier temps dans une solution de chlorhexidine (0,04 g/L). Dans un second temps, les membranes chargées en chlorhexidine ont été rincées à l'eau puis plongées dans un milieu physiologique maintenu à 37° sous agitation. On a ensuite mesuré l'évolution de la concentration en chlorhexidine libérée dans le milieu par spectrophotométrie UV-visible à 255 nm.

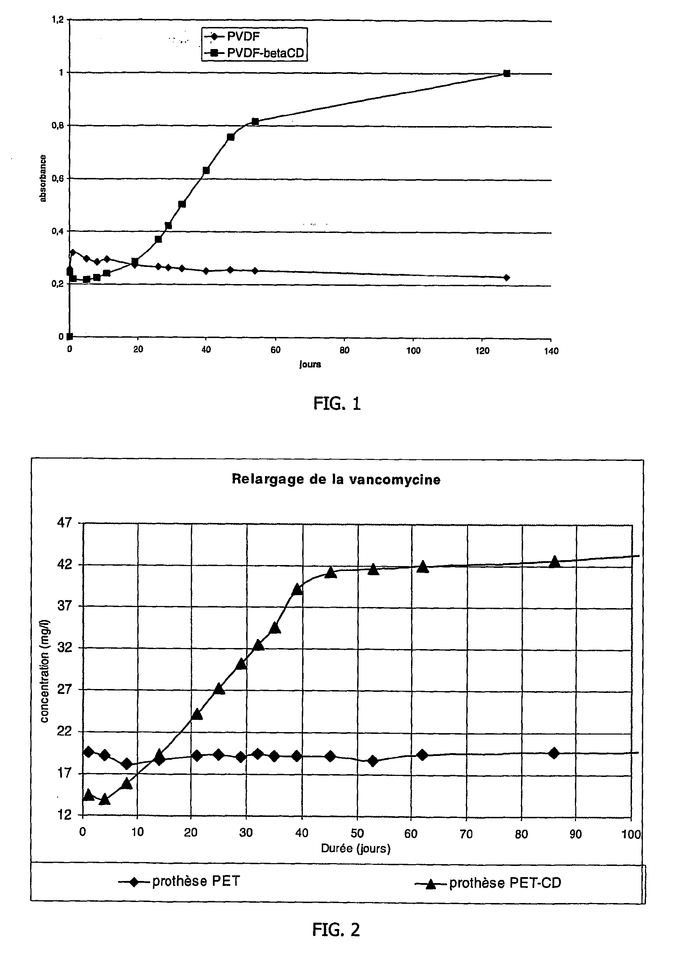

La figure 1 annexée représente l'évolution de la quantité de chlorhexidine libérée dans le milieu par les deux membranes, pendant une durée de 130 jours.

### EXEMPLE 23

Cet exemple illustre les capacités d'absorption accrue et de libération retardée d'une prothèse vasculaire en PET tissé fonctionnalisée par la β-CD vis-à-vis de la Vancomycine, antibiotique choisi comme principe actif. Une prothèse vierge et une seconde fonctionnalisée par la β-CD avec un taux de greffage 10 % ont été plongées dans un premier temps dans une solution de Vancomycine (5 g/L). Dans un second temps, les membranes chargées en Vancomycine ont été rincées à l'eau puis plongées dans un milieu physiologique maintenu à 37° sous agitation. On a ensuite mesuré l'évolution de la concentration en vancomycine dans le milieu par spectrophotométrie UV-visible à 280 nm.

La figure 2 annexée représente l'évolution de la concentration de Vancomycine (en mg/L) libérée dans le milieu par les deux prothèses, pendant une durée de 86 jours.

### EXEMPLE 24

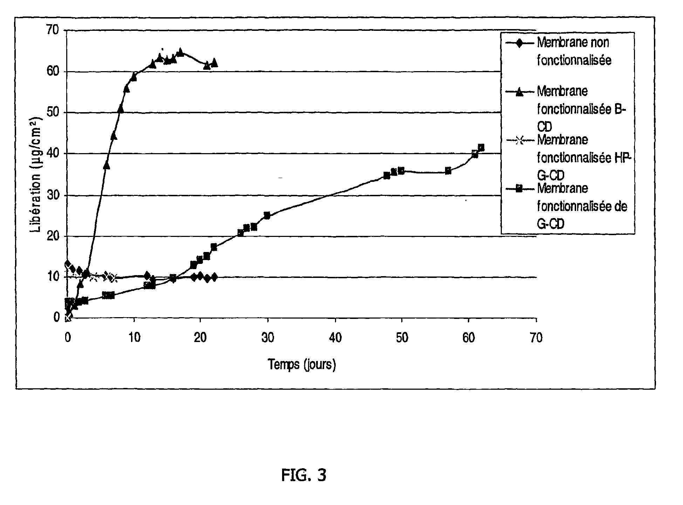

Cet exemple illustre la possibilité de moduler la cinétique de libération d'un agent bioactif, en utilisant un mélange de plusieurs cyclodextrines lors du greffage sur le biomatériau de base et en ajustant la proportion de chaque cyclodextrine dans ce mélange. La figure 3 annexée représente l'étude de libération de la chlorhexidine par des membranes microporeuses de PVDF fonctionnalisées par trois cyclodextrines différentes : β-cylodextrine, hydroxypropyl-γ-cyclodextrine et γ-cydodextrine. Les résultats obtenus montrent que la chlorhexidine est libérée dans les premiers dix jours quand la membrane est greffée de γ-cyclodextrine ; ce délai est de plus de 60 jours pour la β-cylodextrine. En revanche, la hydroxypropyl-γ-cyclodextrine est inefficace puisqu'elle présente le même résultat que la membrane vierge qui libère tout immédiatement, servant de témoin.

## Revendications

1. Procédé de préparation d'un biomatériau comportant au moins une molécule bioactive à partir d'un biomatériau de base **caractérisé par** les opérations successives suivantes effectuées sur ledit biomatériau de base:
a) application d'un mélange solide de :
- cyclodextrine(s) et/ou de dérivé(s) de cyclodextrine(s) et/ou de complexe(s) d'inclusion de cyclodextrine(s) et/ou de dérivés de cyclodextrine(s),
- d'au moins un acide poly(carboxylique),
- et éventuellement d'un catalyseur;
b) chauffage à une température comprise entre 100°C et 220°C, pendant une durée de 1 à 60 minutes ;
c) lavage à l'eau;
d) séchage,
et en ce qu'au moins un agent bioactif est incorporé au biomatériau, par imprégnation du biomatériau après l'étape de séchage dans une solution concentrée d'agent bioactif.

2. Procédé selon la revendication 1, **caractérisé en ce que** l'application du mélange solide est obtenue par imprégnation du biomatériau de base avec une solution aqueuse :
- de cyclodextrine(s) et/ou de dérivé(s) de cyclodextrine(s) ou de complexes de cyclodextrines, ou de complexes d'inclusion cyclodextrine - agent bioactif,
- d'au moins un acide poly(carboxylique),
- et éventuellement d'un catalyseur,
et par séchage du biomatériau de base imprégné.

3. Procédé selon l'une des revendications 1 ou 2, **caractérisé en ce que** le biomatériau de base est séché à une température comprise entre 40°C et 90°C, de préférence, avant l'opération de chauffage proprement dite réalisée à une température comprise entre 100°C et 220°c.

4. Procédé selon l'une quelconque des revendications 1 à 3 **caractérisé en ce qu'**il comporte en outre une étape d'imprégnation du biomatériau, après l'étape de séchage, dans une solution d'agent biocide cationique.

5. Procédé selon l'une quelconque des revendications 1 à 4, **caractérisé en ce que** le biomatériau de base est constitué de matière polymère, tel que le polytéréphtalate d'éthylène glycol (PET), l'acide polylactique, l'acide polyglycolique et leurs copolymères, le fluorure de polyvinylidène (PVDF), le polytétrafluoroéthylène (PTFE), la cellulose, la cellulose oxydée, la cellulose régénérée, le polyéthylène glycol (PEG), le polyamide-6, le polyamide-6,6, le polypropylène, le polyéthylène, la kératine, le chitosane, le collagène ou la gélatine ou de la combinaison de ces polymères.

6. Procédé selon l'une quelconque des revendications 1 à 5, **caractérisé en ce que** le biomatériau de base est constitué de composés minéraux tels que les biocéramiques : phosphates de calcium, hydroxyapatites, alumine, zircone, verres, verres ionomères ou d'un matériau composite à base de composé minéral et de matière polymère.

7. Procédé selon l'une quelconque des revendications précédentes, **caractérisé en ce que** l'acide poly(carboxylique) est choisi parmi les acides poly(carboxyliques) acycliques saturés et insaturés cycliques saturés et insaturés aromatiques, les acides hydroxypoly(carboxyliques), de préférence, l'acide citrique, l'acide polyacrylique, l'acide poly(méthacrylique), l'acide 1, 2, 3, 4-butanetétracarboxylique, l'acide maléique, l'acide citraconique, l'acide itaconique, l'acide 1, 2, 3-propanetricarboxylique, l'acide trans-aconitique, l'acide all-cis-1, 2, 3, 4-cydopentanetétracarboxylique, l'acide méllittique, l'acide oxydisuccinique, l'acide thiodisuccinique, ou parmi les anhydrides dérivés des acides sus nommés.

8. Procédé selon l'une quelconque des revendications précédentes, **caractérisé en ce que** le catalyseur est choisi parmi les dihydrogénophosphates, les hydrogénophosphates, les phosphates, les hypophosphites, les phosphites de métaux alocalins, les sels de métaux alcalins des acides polyphosphoriques, les carbonates, les bicarbonates, les acétates, les borates, les hydroxydes de métaux alcalins, les amines aliphatiques et l'ammoniaque, et de préférence, parmi l'hydrogénophosphate de sodium, le dihydrogénophosphate de sodium et l'hypophosphite de sodium.

9. Procédé selon l'une quelconque des revendications précédentes, **caractérisé en ce que** la cyclodextrine est choisie parmi : l'α-cyclodextrine, la β-cyclodextrine la γ-cyclodextrine et leurs mélanges ; leurs dérivés qui sont choisis préférentiellement parmi les dérivés aminés, méthylés, hydroxypropylés, sulfobutylés, carboxyméthylés, ou carboxyliques de ces cyclodextrines, et leurs mélanges.

10. Procédé selon l'une quelconque des revendications précédentes **caractérisé en ce que** ledit agent bioactif est sélectionné parmi les anticoagulants, les anti-thrombogéniques, les agents anti-mitotiques, les agents anti-prolifération, anti-adhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques ou les antibiotiques.

11. Biomatériau préparé de préférence selon le procédé de l'une quelconque des revendications 1 à 10 à partir d'un biomatériau de base présentant une structure chimique qui comporte une fonction hydroxyle et/ou une fonction amine, ladite structure étant liée de façon covalente à au moins une molécule de cyclodextrine ou à un polymère composé de cyclodextrine dont la structure comporte la répétition du motif de formule générale : 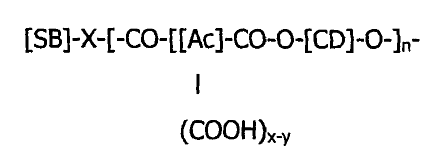 SB représentant la structure du biomatériau de base constitué d'un matériau polymère d'origine naturelle ou artificielle et/ou d'un matériau biocéramique, X étant soit un atome d'oxygène soit un groupe NH,
x ≥ 3, et 2≤y ≤(x-1) et (x-y) ≥1,
- x étant le nombre de fonctions carboxyle portées par l'acide poly(carboxylique) avant réaction,
- y représente le nombre de fonctions carboxyle de l'acide poly(carboxylique) estérifiées ou amidifiées lors de la réaction,
- (x-y) est le nombre de fonctions carboxyle de l'acide poly(carboxylique) non estérifiées ou amidifiées après réaction,
- 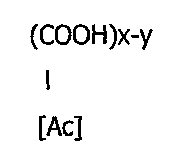 représentant la chaîne moléculaire d'un acide poly(carboxylique) de formule 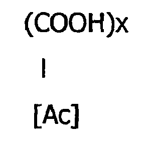 dont au moins deux fonctions carboxyle (-COOH) ont subi une estérification ou subi respectivement une estérification et une amidification et qui porte au moins une fonction carboxyle n'ayant pas subi de réaction d'estérification ou d'amidification ;
- [CD] représentant la structure moléculaire des cyclodextrines choisies préférentiellement parmi : l'α-cyclodextrine, la β-cyclodextrine, la γ-cyclodextrine et leurs mélanges; leurs dérivés qui sont choisis préférentiellement parmi les dérivés aminés, méthylés, hydroxypropylés, sulfobutylés, carboxyméthylés, ou carboxyliques, et leurs mélanges,
**caractérisé en ce que**, ledit biomatériau comportant au moins un agent bioactif formant un complexe avec les molécules de cyclodextrine et/ou de dérivés de cyclodextrine, ledit agent bioactif est une molécule thérapeutique.

12. Biomatériau préparé de préférence selon le procédé de l'une quelconque des revendications 1 à 10 à partir d'un biomatériau de base dont la structure poreuse est occupée, ou la surface de la structure est enrobée ou recouverte par un polymère réticulé de cyclodextrine(s) et/ou de dérivés de cyclodextrine(s) et/ou de complexes d'inclusion de cyclodextrine ou de dérivés de cyclodextrines et dont la structure comporte la répétition d'un motif de formule générale : 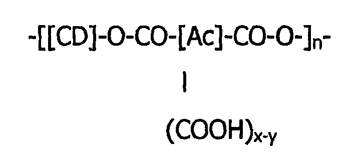 avec x ≥ 3, et 2≤y ≤(x-1) et (x-y) ≥1,
- x étant le nombre de fonctions carboxyle portées par l'acide poly(carboxylique) avant réaction,
- y représentant le nombre de fonctions carboxyle de l'acide poly(carboxylique) estérifiées lors de la réaction,
- (x-y) étant le nombre de fonctions carboxyle de l'acide poly(carboxylique) non estérifiées après réaction
- 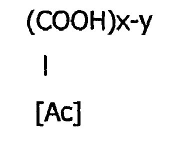 représentant la chaîne moléculaire d'un acide poly(carboxylique) de formule : 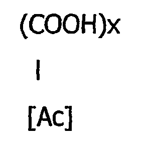 dont au moins deux fonctions carboxyle (-COOH) ont subi une estérification et qui porte au moins une fonction carboxyle n'ayant pas subi de réaction d'estérification;
- [CD] représentant la structure moléculaire des cyclodextrines choisies préférentiellement parmi: l'α-cyclodextrine, la β-cyclodextrine, la γ-cyclodextrine et leurs mélanges; leurs dérivés qui sont choisis préférentiellement parmi les dérivés aminés, méthylés, hydroxypropylés, sulfobutylés, carboxyméthylés, ou carboxyliques, et leurs mélanges,
**caractérisé en ce que**, ledit biomatériau comportant au moins un agent bioactif formant un complexe avec les molécules de cyclodextrine et/ou de dérivés de cyclodextrine, ledit agent bioactif est une molécule thérapeutique.

13. Biomatériaux selon l'une quelconque des revendications 11 et 12, **caractérisé en ce que** le biomatériau de base est constitué de matière polymère, tel que le polytéréphtalate d'éthylène glycol (PET) l'acide polylactique, l'acide polyglycolique et leurs copolymères, le fluorure de polyvinylidène (PVDF), le polytétrafluoroéthylène (PTFE), la cellulose, la cellulose oxydée, la cellulose régénérée, le polyéthylène glycol (PEG), le polyamide-6, le polyamide-6,6, le polypropylène, le polyéthylène, les polysaccharides tels que pectine, carraghénanes, alginates et dextranes, la kératine, le chitosane, le collagène ou la gélatine ou de la combinaison de ces polymères.

14. Biomatériaux selon l'une quelconque des revendications 11 et 12, **caractérisé en ce que** le biomatériau de base est constitué de composés minéraux tels que les biocéramiques : phosphates de calcium, hydroxyapatites, alumine, zircone, verres, verres ionomères ou d'un matériau composite à base de composé minéral et de matière polymère.

15. Biomatériau selon l'une des revendications 11 à 14 **caractérisé en ce que** l'agent bioactif est choisi parmi les anticoagulants, les anti-thrombogéniques, les agents anti-mitotiques, les agents anti-prolifération, anti-adhésion, anti-migration, les promoteurs d'adhésion cellulaire, les facteurs de croissance, les molécules antiparasitaires, anti-inflamatoires, les antifongiques, les molécules antimicrobiennes, les antiseptiques et les antibiotiques.

16. Utilisation d'un biomatériau tel que défini par l'une quelconque des revendications 11 à 15 pour la préparation d'un implant, d'une prothèse ou d'un dispositif pour la libération contrôlée d'au moins une molécule thérapeutique.

17. Utilisation d'un biomatériau selon la revendication 16 **caractérisée en ce que** le biomatériau exerce la fonction de prothèse et endoprothèse vasculaire (stent couvert), de plaque de contention de hernies, de membrane de régénération tissulaire guidée, de membrane de régénération osseuse guidée, ou de dispositif de libération intrasucculaires, de tube et cathéter de dialyse, de perfusion, de transfusion, de nutrition artificielle, d'implant transcutanés, de treillis et réseau pour ingénierie tissulaire, de substitut osseux micro et macroporeux, de fil de suture et de pansement à usage médical et vétérinaire.

18. Dispositif pour la libération contrôlée d'au moins une molécule thérapeutique **caractérisé par le fait qu'**il comprend au moins un biomatériau tel que défini par l'une quelconque des revendications 11 à 15.

19. Composition **caractérisée par le fait qu'**elle renferme au moins un biomatériau tel que défini par l'une quelconque des revendications 11 à 15.

## Claims

1. A method of preparation of a biomaterial, containing at least one bioactive molecule from a base biomaterial, **characterised by** the following successive operations carried out on said base biomaterial:
a) application of a solid mixture of:
- cyclodextrin(s) and/or cyclodextrin derivative(s) and/or cyclodextrin inclusion complex(es) and/or cyclodextrin derivative inclusion complex(es),
- at least one poly(carboxylic) acid, and
- optionally a catalyst;
b) heating at a temperature between 100 °C and 220 °C for a period of 1 to 60 minutes;
c) washing with water;
d) drying,
and in that at least one bioactive agent is incorporated in the biomaterial by impregnation of the biomaterial after the drying step in a concentrated solution of bioactive agent.

2. A method according to claim 1, **characterised in that** the application of the solid mixture is obtained by impregnation of the base biomaterial with an aqueous solution of:
- cyclodextrin(s) and/or cyclodextrin derivative(s) or cyclodextrin inclusion complexes or cyclodextrin-bioactive agent inclusion complexes,
- at least one poly(carboxylic) acid, and
- optionally a catalyst,
by drying the impregnated base biomaterial.

3. A method according to claim 1 or claim 2, **characterised in that** the base biomaterial is dried at a temperature between 40 °C and 90 °C, preferably, before the primary heating operation which is carried out at a temperature between 100 °C and 220 °C.

4. A method according to any one of claims 1 to 3, **characterised in that** it contains in addition a step of impregnation of the biomaterial in a solution of cationic biocidal agent, after the drying step.

5. A method according to any one of claims 1 to 4, **characterised in that** the base biomaterial consists of polymer material such as polyethylene terephthalate glycol (PETG), polylactic acid, polyglycolic acid and copolymers thereof, polyvinylidene fluoride (PVDF), polytetrafluoroethylene (PTFE), cellulose, oxidized cellulose, regenerated cellulose, polyethylene glycol (PEG), polyamide-6, polyamide-6,6, polypropylene, polyethylene, keratin, chitosan, collagen, gelatin or a combination of said polymers.

6. A method according to any one of claims 1 to 5, **characterised in that** the base biomaterial is comprised of mineral compounds such as bioceramics, including calcium phosphates, hydroxyapatites, alumina, zirconia, glass, ionomer glass or a composite material containing a mineral compound and a polymer material.

7. A method according to any one of the preceding claims, **characterised in that** the poly(carboxylic) acid is selected among acyclic poly(carboxylic) acids, saturated and unsaturated cyclic poly(carboxylic) acids, saturated and unsaturated aromatic poly(carboxylic) acids, hydroxypoly(carboxylic)acids, preferably citric acid, polyacrylic acid, poly(methacrylic) acid, 1,2,3,4-butanetetracarboxylic acid, maleic acid, citraconic acid, itaconic acid, 1,2,3-propanetricarboxylic acid, trans-aconitic acid, all-cis-1,2,3,4-cydopentanetetracarboxylic acid, mellitic acid, oxydisuccinic acid, thiodisuccinic acid or among anhydride derivatives of aforesaid acids.

8. A method according to any one of the preceding claims, **characterised in that** the catalyst is selected among dihydrogen phosphates, hydrogen phosphates, phosphates, hypophosphites, alkaline metal phosphites, polyphosphoric acid alkaline metal salts, carbonates, bicarbonates, acetates, borates, alkaline metal hydroxides, aliphatic and ammonia amines, preferably among sodium hydrogen phosphate, sodium dihydrogen phosphate and sodium hypophosphite.

9. A method according to any one of the preceding claims, **characterised in that** the cyclodextrin is selected among: α-cyclodextrin, β-cyclodextrin, γ-cyclodextrin and mixtures thereof; derivatives thereof preferentially selected among the amino, methyl, hydroxypropyl, sulfobutyl, carboxymethyl or carboxyl derivatives of said cyclodextrins, and mixtures thereof.

10. A method according to any one of receding claims, **characterised in that** the aforesaid bioactive agent is selected among anticoagulants, antithrombotics, antimitotics, antiproliferation agents, antiadhesion agents, antimigration agents, cell adhesion promoters, growth factors, antiparasitic molecules, anti-inflammatories, anti-angiogenics, angiogenesis inhibitors, vitamins, hormones, proteins, antifungals, antimicrobial molecules, antiseptics or antibiotics.

11. Biomaterial prepared from a base biomaterial, preferably according to the method of any one of claims 1 to 10, having a chemical structure which contains a hydroxyl function and/or an amine function, said structure being bound covalently to at least one molecule of cyclodextrin or to a polymer comprised of cyclodextrin whose structure contains the repetition of the unit of general formula: 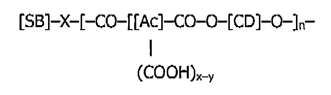 SB representing the structure of the base biomaterial comprised of a polymer material of natural or artificial origin and/or of a bioceramic material, X being either an oxygen atom or an NH group,
with x≥3, 2≤y≤(x-1) and (x-y)≥1,
- x being the number of carboxyl functions carried by the poly(carboxylic) acid before reaction,
- y representing the number of carboxyl functions of the poly(carboxylic) acid formed into esters or amides during the reaction,
- (x-y) being the number of carboxyl functions of the poly(carboxylic) acid not formed into esters or amides after reaction,
- 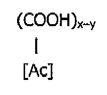 representing the molecular chain of a poly(carboxylic) acid of formula: 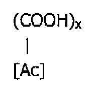 of which at least two carboxyl functions (-COOH) have undergone esterification or, respectively, esterification and amide formation and which carries at least one carboxyl function not having undergone an esterification or amide formation reaction;
- [CD] representing the molecular structure of the cyclodextrins chosen preferentially among: α-cyclodextrin, β-cyclodextrin, γ-cyclodextrin and mixtures thereof; derivatives thereof which are preferentially selected among amino, methyl, hydroxypropyl, sulfobutyl, carboxymethyl or carboxyl derivatives and mixtures thereof,
**characterised in that** the said biomaterial containing at least one bioactive agent forming a complex with the molecules of cyclodextrin and/or of cyclodextrin derivatives, the said bioactive agent is a therapeutic molecule.

12. Biomaterial prepared from a base biomaterial, preferably according to the method of any one of claims 1 to 10, whose porous structure is occupied by, or the surface structure is coated or covered by a crosslinked polymer of cyclodextrin(s) and/or cyclodextrin derivative(s) and/or cyclodextrin inclusion complexes or cyclodextrin derivative inclusion complexes and whose structure contains the repetition of the unit of general formula: 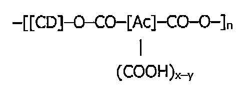 with x ≥ 3, 2 ≤ y ≤ (x-1) and (x-y) ≥ 1,
- x being the number of carboxyl functions carried by the poly(carboxylic) acid before reaction,
- y representing the number of carboxyl functions of the poly(carboxylic) acid esterified during the reaction,
- (x-y) being the number of carboxyl functions of the poly(carboxylic) acid not esterified after reaction,
- 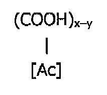 representing the molecular chain of a poly(carboxylic) acid of formula: 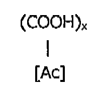 of which at least two carboxyl functions (-COOH) have undergone esterification and which carries at least one carboxyl function not having undergone an esterification reaction;
- [CD] representing the molecular structure of the cyclodextrins chosen preferentially among: α-cyclodextrin, β-cyclodextrin, γ-cyclodextrin and mixtures thereof; derivatives thereof which are preferentially selected among amino, methyl, hydroxypropyl, sulfobutyl, carboxymethyl or carboxyl derivatives and mixtures thereof,
**characterised in that**, the aforesaid biomaterial containing at least one bioactive agent forming a complex with the molecules of cyclodextrin and/or of cyclodextrin derivatives, the said bioactive agent is a therapeutic molecule.

13. Biomaterials according to claim 11 or claim 12, **characterised in that** the base biomaterial consists of polymer material such as polyethylene terephthalate glycol (PETG), polylactic acid, polyglycolic acid and copolymers thereof, polyvinylidene fluoride (PVDF), polytetrafluoroethylene (PTFE), cellulose, oxidized cellulose, regenerated cellulose, polyethylene glycol (PEG), polyamide-6, polyamide-6,6, polypropylene, polyethylene, polysaccharides such as pectin, carrageenans, alginates and dextrans, keratin, chitosan, collagen, gelatin or a combination of said polymers.

14. Biomaterials according to claim 11 or claim 12, **characterised in that** the base biomaterial consists of mineral compounds such as bioceramics, including calcium phosphates, hydroxyapatites, alumina, zirconia, glass, ionomer glass or a composite material containing a mineral compound and a polymer material.

15. Biomaterial according to one of claims 11 to 14, **characterised in that** the bioactive agent is selected among anticoagulants, antithrombotics, antimitotics, antiproliferation agents, antiadhesion agents, antimigration agents, cell adhesion promoters, growth factors, antiparasitic molecules, anti-inflammatories, antifungals, antimicrobial molecules, antiseptics or antibiotics.

16. Use of a biomaterial as defined by any one of claims 11 to 15 for the preparation of an implant, a prosthesis or a device for the controlled release of at least one therapeutic molecule.

17. Use of a biomaterial according to claim 16, **characterised in that** the biomaterial has the function of a vascular prosthesis or endoprosthesis (coated stent); a hernia support; a guided tissue regeneration membrane; a guided bone regeneration membrane; an intrasulcular release device; a dialysis, perfusion, transfusion or artificial nutrition tube or catheter; a transcutaneous implant; a tissue engineering lattice or network; a micro-or macro-porous bone substitute; or suture or bandage wire or thread for medical or veterinary use.

18. A device for the controlled release of at least one therapeutic molecule, **characterised by** the fact that said device comprise at least one biomaterial as defined by any one of claims 11 to 15.

19. A composition **characterised by** the fact that it contains at least one biomaterial as defined by any one of claims 11 to 15.

## Patentansprüche

1. Verfahren zur Herstellung eines Biomaterials, das wenigstens ein bioaktives Molekül umfaßt, aus einem Ausgangsbiomaterial, **gekennzeichnet durch** die folgenden aufeinanderfolgenden Vorgänge, die auf dem Ausgangsbiomaterial ausgeführt werden:
a) Aufbringen eines festen Gemischs aus:
- Cyclodextrin(en) und/oder Cyclodextrinderivat(en) und/oder Cyclodextrineinschlußkomplex(en) und/oder Cyclodextrinderivat(en),
- wenigstens einer Polycarboxylsäure,
- und gegebenenfalls einem Katalysator,
b) Erhitzen bei einer Temperatur zwischen 100°C und 220°C für eine Dauer von 1 bis 60 Minuten,
c) Waschen mit Wasser,
d) Trocknen,
und **dadurch**, daß wenigstens ein bioaktives Mittel dem Biomaterial einverleibt wird, **durch** Imprägnieren des Biomaterials nach dem Trocknungsschritt in einer konzentrierten Lösung des bioaktiven Mittels.

2. Verfahren nach Anspruch 1, **dadurch gekennzeichnet, daß** das Aufbringen des festen Gemischs erhalten wird durch Imprägnieren des Ausgangsbiomaterials mit einer wäßrigen Lösung:
- von Cyclodextrin(en) und/oder Cyclodextrinderivat(en) oder Cyclodextrinkomplexen oder Cyclodextrin-Bioaktivmittel-Einschlußkomplexen,
- wenigstens einer Polycarboxylsäure,
- und gegebenenfalls einem Katalysator
und durch Trocknen des imprägnierten Ausgangsbiomaterials.

3. Verfahren nach einem der Ansprüche 1 oder 2, **dadurch gekennzeichnet, daß** das Ausgangsbiomaterial getrocknet wird bei einer Temperatur zwischen 40°C und 90°C, vorzugsweise, vor dem genannten Erhitzungsvorgang, der ausgeführt wird bei einer Temperatur zwischen 100°C und 200°C.

4. Verfahren nach einem der Ansprüche 1 bis 3, **dadurch gekennzeichnet, daß** es außerdem einen Imprägnierungsschritt des Biomaterials nach dem Trocknungsschritt in einer Lösung von kationischem bioziden Mittel umfaßt.

5. Verfahren nach einem der Ansprüche 1 bis 4, **dadurch gekennzeichnet, daß** das Ausgangsbiomaterial aus Polymermaterial besteht wie Polyethylenterephthalat (PET), Polymilchsäure, Polyglycolsäure und deren Copolymere, Polyvinylidenfluorid (PVDF), Polytetrafluorethylen (PTFE), Zellulose, oxidierte Zellulose, regenerierte Zellulose, Polyethylenglycol (PEG), 6-Polyamid, 6,6-Polyamid, Polypropylen, Polyethylen, Keratin, Chitosan, Kollagen oder Gelatine oder die Kombination dieser Polymere.

6. Verfahren nach einem der Ansprüche 1 bis 5, **dadurch gekennzeichnet, daß** das Ausgangsbiomaterial besteht aus Mineralverbindungen wie den Biokeramiken: Kalziumphosphate, Hydroxyapatite, Aluminiumoxid, Zirconiumoxid, Gläser, Glasionomere oder Gläser aus einem Verbundmaterial auf Basis einer mineralischen Verbindung und Polymermaterial.

7. Verfahren nach einem der vorhergehenden Ansprüche, **dadurch gekennzeichnet, daß** die Polycarboxylsäure gewählt wird unter den azyklischen gesättigten und ungesättigten, zyklischen gesättigten und ungesättigten aromatischen Polycarboxylsäuren, den Hydroxypolycarboxylsäuren, vorzugsweise Zitronensäure, Polyacrylsäure, Polymethacrylsäure, 1,2,3,4-Butantetracarboxylsäure, Maleinsäure, Citraconsäure, Itaconsäure, 1,2,3-Propantricarboxylsäure, trans-Aconitinsäure, All-cis-1,2,3,4-Cyclopentantetracarboxlsäure, Mellitsäure, Oxydisuccinsäure, Thiodisuccinsäure oder unter den Anhydridderivaten der oben genannten Säuren.

8. Verfahren nach einem der vorhergehenden Ansprüche, **dadurch gekennzeichnet, daß** der Katalysator gewählt wird unter den Dihydrogenphosphaten, den Hydrogenphosphaten, den Phosphaten, den Hypophosphiten, den Alkalimetallphosphiten, den Alkalimetallsalzen der Polyphosphorsäuren, den Carbonaten, den Bicarbonaten, den Acetaten, den Boraten, den Alkalimetallhydroxiden, den aliphatischen Aminen und Ammoniak und vorzugsweise unter Natriumhydrogenphosphat, Natriumdihydrogenphosphat und Natriumhypophosphit.

9. Verfahren nach einem der vorhergehenden Ansprüche, **dadurch gekennzeichnet, daß** das Cyclodextrin gewählt ist unter: α-Cyclodextrin, β-Cyclodextrin, γ-Cyclodextrin und deren Gemischen, deren Derivaten, die vorzugsweise gewählt sind unter den aminierten, methylierten, hydroxypropylierten, sulfobutylierten, carboxymethylierten Derivaten oder Carboxylderivaten dieser Cyclodextrine und deren Gemischen.

10. Verfahren nach einem der vorhergehenden Ansprüche, **dadurch gekennzeichnet, daß** das bioaktive Mittel gewählt ist unter den Anticoagulaten, den Antithrombogenen, den antimitotischen Mitteln, den Antiproliferationsmitteln, den Antiadhäsionsmitteln, den Antimigrationsmitteln, den Zelladhäsionspromotoren, den Wachstumsfakturen, den antiparasitären Molekülen, den Antientzündungsmitteln, den angiogenischen Mitteln, den Angiogeneseinhibitoren, den Vitaminen, den Hormonen, den Proteinen, den Fungiziden, den antimikrobiellen Molekülen, den Antiseptika oder den Antibiotika.

11. Biomaterial, vorzugsweise hergestellt nach einem Verfahren nach einem der Ansprüche 1 bis 10, aus einem Ausgangsbiomaterial, das eine chemische Struktur aufweist, die eine Hydroxylfunktion und/oder eine Aminfunktion umfaßt, wobei die Struktur covalent gebunden ist an wenigstens ein Cyclodextrinmolekül oder an eine Cyclodextrinpolymerverbindung, deren Struktur die Wiederholung des allgemeinen Motivs umfaßt: 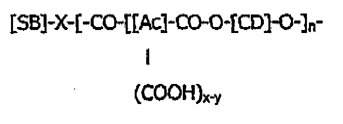 wobei SB die Struktur des Ausgangsbiomaterials darstellt, das besteht aus einem Polymermaterial natürlichen oder künstlichen Ursprungs und/oder einem biokeramischen Material, wobei X entweder ein Sauerstoffatom oder eine NH-Gruppe ist,
x ≥ 3 und 2 ≤ y ≤ (X-1) und (x-y) ≥ 1,
- wobei x die Anzahl von Carboxylfunktionen ist, die getragen sind durch die Polycarboxylsäure vor Reaktion,
- wobei y die Anzahl von Carboxylfunktionen der Polycarboxylsäure ist, die esterifiziert oder amidifiziert sind bei der Reaktion,
- (x-y) die Anzahl der Carboxylfunktionen der Polycarboxylsäure ist, die nicht esterifiziert oder amidifiziert sind nach Reaktion, wobei
- 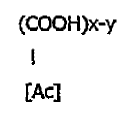 eine Molekülkette einer Polycarboxylsäure darstellt mit Formel 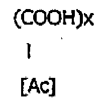 von der wenigstens zwei Carboxylfunktionen (-COOH) einer Esterifizierung unterlagen oder jeweils einer Esterifizierung und einer Amidifizierung unterlagen und welche wenigstens eine Carboxylfunktion umfaßt, die keiner Esterifizierungs- oder Amidifizierungsreaktion unterlegen ist,
- wobei [CD] eine Molekülstruktur der Cyclodextrine darstellt, vorzugsweise gewählt unter: α-Cyclodextrin, β-Cyclodextrin, γ-Cyclodextrin und deren Gemischen, deren Derivaten, die vorzugsweise gewählt sind unter den aminierten, methylierten, hydroxypropylierten, sulfobutylierten, carboxymethylierten Derivaten oder Carboxylderivaten und deren Gemischen, **dadurch gekennzeichnet, daß**, da das Biomaterial wenigstens ein bioaktives Mittel umfaßt, das einen Komplex mit den Cyclodextrinmolekülen und/oder Cyclodextrinderivaten bildet, wobei das bioaktive Mittel ein therapeutisches Molekül ist.

12. Biomaterial, vorzugsweise hergestellt nach einem Verfahren nach einem der Ansprüche 1 bis 10, aus einem Ausgangsbiomaterial, dessen poröse Struktur besetzt ist oder die Oberfläche der Struktur eingekleidet oder bedeckt ist durch ein vernetztes Polymer von Cyclodextrin(en) und/oder Cyclodextrinderivat(en) und/oder Cyclodextrineinschlußkomplex(en) oder Cyclodextrinderivaten und deren Struktur die Wiederholung eines Motivs mit allgemeiner Formel umfaßt: 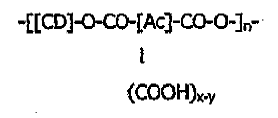 mit x ≥ 3 und 2 ≤ y ≤ (x-1) und (X-y) ≥ 1,
- wobei x die Anzahl der Carboxylfunktionen ist, die durch die Polycarboxylsäure vor Reaktion getragen sind,
- wobei y die Anzahl der Carboxylfunktionen der Polycarboxylsäure darstellt, die bei der Reaktion esterifiziert sind,
- wobei (x-y) die Anzahl der Carboxylfunktionen der Polycarboxylsäure ist, die nach Reaktion nicht esterifiziert sind, wobei
- 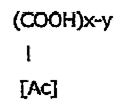 eine Molekülkette einer Polycarboxylsäure darstellt mit Formel: 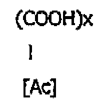 von der wenigstens zwei Carboxylfunktionen (-COOH) einer Esterifizierung unterlegen sind und welche wenigstens eine Carboxylfunktion umfassen, die keiner Esterifizierungsreaktion unterlegen ist,
- wobei [CD] eine Molekülstruktur der Cyclodextrine darstellt, vorzugsweise gewählt unter: α-Cyclodextrin, β-Cyclodextrin, γ-Cyclodextrin und deren Gemischen, deren Derivaten, die vorzugsweise gewählt sind unter den aminierten, methylierten, hydroxypropylierten, sulfobutylierten, carboxymethylierten Derivaten oder Carboxylderivaten und deren Gemischen, **dadurch gekennzeichnet, daß**, da das Biomaterial wenigstens ein bioaktives Mittel umfaßt, das einen Komplex mit den Cyclodextrinmolekülen und/oder Cyclodextrinderivaten bildet, wobei das bioaktive Mittel ein therapeutisches Molekül ist.

13. Biomaterialien nach einem der Ansprüche 11 und 12, **dadurch gekennzeichnet, daß** das Ausgangsbiomaterial aus Polymermaterial besteht wie Ethylenpolyterephtalat (PET), Polymilchsäure, Polyglycolsäure und deren Copolymere, Polyvinylidenfluorid (PVDF), Polytetrafluorethylen (PTFE), Zellulose, oxidierte Zellulose, regenerierte Zellulose, Polyethylenglycol (PEG), 6-Polyamid, 6,6-Polyamid, Polypropylen, Polyethylen, den Polysacchariden wie Pektin, Carraghenane, Alginate und Dextrane, Keratin, Chitosan, Kollagen oder Gelatine oder die Kombination dieser Polymere.

14. Biomaterialien nach einem der Ansprüche 11 und 12, **dadurch gekennzeichnet, daß** das Ausgangsbiomaterial besteht aus Mineralverbindungen wie den Biokeramiken: Kalziumphosphate, Hydroxyapatite, Aluminiumoxid, Zirconiumoxid, Gläser, Glasionomere oder Gläser aus einem Verbundmaterial auf Basis einer mineralischen Verbindung und Polymermaterial.

15. Biomaterial nach einem der Ansprüche 11 bis 14, **dadurch gekennzeichnet, daß** das bioaktive Mittel gewählt ist unter den Anticoagulaten, den Antithrombogenen, den antimitotischen Mitteln, den Anliproliferationsmitteln, den Antiadhäsionsmitteln, den Antimigrationsmitteln, den Zelladhäsionspromotoren, den Wachstumsfakturen, den antiparasitären Molekülen, den Antientzündungsmitteln, den Fungiziden, den antimikrobiellen Molekülen, den Antiseptika oder den Antibiotika.

16. Verwendung eines Biomaterials wie definiert in einem der Ansprüche 11 bis 15 zur Herstellung eines Implantats einer Prothese oder einer Vorrichtung für die kontrollierte Freisetzung wenigstens eines therapeutischen Moleküls.

17. Verwendung eines Biomaterials nach Anspruch 16, **dadurch gekennzeichnet, daß** das Biomaterial die Funktion einer Prothese und Gefäßendoprothese ausführt (bedecktes Stent), einer Bandscheibenhalteplatte, einer geführten Geweberegenerationsmembran, die Funktion einer geführten Knochenregenerationsmembran oder einer Vorrichtung zur intrasukkularen Freisetzung, die Funktion eines Röhrchens und Katheters zur Dialyse, Perfusion, Transfusion, künstliche Ernährung, transkutaner Implantate, eines Geflechts und Netzwerks zum Gewebe-Engineering, eines mikro- und makroporösen Knochenersatzes, eines Naht- und Verbandsfadens zur medizinischen und veterinären Verwendung ausführt.

18. Vorrichtung zur kontrollierten Freisetzung wenigstens eines therapeutischen Moleküls, **gekennzeichnet durch** die Tatsache, daß sie wenigstens ein Biomaterial wie definiert nach einem der Ansprüche 11 bis 15 umfaßt.

19. Zusammensetzung, **dadurch gekennzeichnet, daß** sie wenigstens ein Biomaterial wie definiert in einem der Ansprüche 11 bis 15 umfaßt.
